# EUROPEAN PATENT SPECIFICATION

(11) **EP 3 056 134 B1**
(45) Date of publication and mention of the grant of the patent: **05.09.2018**
(21) Application number: 16155346.6
(22) Date of filing: 11.02.2016
(51) Int. Cl.: A61B 1/00, A61B 1/307, A61B 17/34, A61M 25/00

(54) **ENDOSCOPE NEEDLE GUIDE FOR INJECTIONS AT AN ANGLE INTO THE WALLS OF TUBULAR ORGANS TO THE PREDETERMINED DEPTH**
ENDOSKOPNADELFÜHRUNG FÜR INJEKTIONEN IN EINEM WINKEL IN DIE WÄNDE TUBULÄRER ORGANE MIT VORBESTIMMTER TIEFE
GUIDE D'AIGUILLE D'ENDOSCOPE POUR INJECTIONS SELON UN ANGLE DANS LES PAROIS D'ORGANES TUBULAIRES À LA PROFONDEUR PRÉDÉTERMINÉE

(30) Priority: 11.02.2015 PL 41120115
(43) Date of publication of application: 17.08.2016
(73) Proprietor: Warszawski Uniwersytet Medyczny, 02-091 Warszawa (PL)
(72) Inventor: Burdzinska, Anna, 04-305 Warszawa (PL); Kaupa, Patryk, 05-509 Józefoslaw (PL); Dybowski, Bartosz, Warszawa (PL)
(74) Representative: Witek, Rafal

(56) References cited:
- WO-A1-99/47069
- US-A1- 2002 143 302
- US-A1- 2006 189 940
- US-A1- 2014 194 776

## Description

Presented invention relates to an endoscope needle guide for injections at an angle and apparatus for injections at an angle applicable in treatments consisting in performing repeatable injections at an angle to the predetermined depth, in particular injections in the area of the urethra, performed with a special type of endoscope - the urethro-cystoscope.

Urinary incontinence is a very common medical and social problem. Numerous epidemiologic researches indicate that urinary incontinence in women in the middle age (from ca.40 to ca.60 years old) oscillates around 25-30%. According to Norton and Brubaker in scientific publication by Norton P.,Brubaker L. "Urinary incontinence in women," Lancet. 2006;367(9504):57-67, due to the populations' getting older as well as lack of simple therapeutic procedures, the costs of care and treatment of patients with urinary incontinence are very high and will be growing further. Periurethral injections are one of the treatment methods (they are aimed at strengthening the force closing the urethra),however, materials used so far did not guarantee satisfactory effectiveness. For about a decade, research on application of stem cells as the new material for injections has been carried out. In scientific publication: Burdzińska A.,Crayton R.,Dybowski B., Idziak M.,Gala K.,Radziszewski P., P 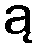 czek L,.,, The effect of endoscopic administration of autologous porcinemuscle-derived cells into the urethral sphincter,"Urology,2013;82(3):743.e1-8, it was demonstrated that the precision of injection of cell suspension can be of key importance for the therapeutic success. The wall of the human urethra is a few millimeters thick, whereas the sphincter is only a fragment of that wall in cross-section. Typically, urethral injections are performed with a urethro-cystoscope, but it does not enable control of injection angle and by the same the control of the depth that the needle is introduced to in the line perpendicular to the longitudinal axis of the urethra.

From international patent application No. WO9616606, a medical device for treatment of urinary incontinence is known, consisting of an elongate member from which at least one needle extends. The needle moves in a channel of a special construction, which provides an appropriate angle of deflection in relation to the longitudinal axis of the medical device. The treatment agent is injected through the needle introduced at the appropriate angle in the urethral area. The presented solution is characterized with complex construction, which makes its precise positioning impossible. There is no possibility to change the needle angle, and specifying the injection depth is substantially obstructed.

International patent application No. WO9913785 discloses a medical device for minimally invasive access to the pericardial space. The device consists of a cylindrical member inside which a needle is moved axially. The device comprises a guide tube, situated at its distal end, wherein the deflecting mechanism is situated. Appropriately designed deflecting mechanism provides angular deflection of the needle guided inside the device, which allows introducing the needle at a predetermined angle. The presented device, however, does not provide appropriate control of the depth of penetration with the needle and is characterized with complex structure.

On the other hand, international patent application WO9210142 relates to a catheter and movable needle system for appropriate placing fiber optic elements and thermo-measuring devices into the bulk of an adjacent organ. In the presented device, the needles driving the fiber optic elements and/or thermo-measuring elements move inside shaped channels providing deflection from the longitudinal axis of the device and cause radial puncture at appropriate angle. The puncture angle is predetermined by the shape of the channels and its alteration requires building another device. Furthermore, the construction of the device does not provide appropriate control of needle penetration depth into the given organ.

European patent No. EP2653184 discloses a medical device for treating stress urinary incontinence. The device comprises a cannula with a plurality of apertures through which a plurality of needles extends. The handle of the device comprises a housing and a shifter mechanism to translate the plurality of needles from a first position (the needles disposed within the cannula) to a second position (needles extending out of the cannula through respective apertures). The handle of the device also comprises a slot and a plurality of longitudinal tracks, which provides additional motion of the shifter mechanism with respect to the housing, making it possible to extend the needles to different distances and providing rotational motion of the shifter mechanism with respect to the housing, which in turn enables changing the puncture position on the perimeter of the tissuein which the cannula is placed. The medical device presented in the patent referred to is of complex structure containing may components and a movable mechanism, which makes its production relatively expensive. Furthermore, the alteration of the puncture angle is possible only upon replacement of the cannula and/or the whole device, which in turn reduces its universality. It also does not allow to preview the performed injections.

Related devices are also disclosed in documents WO 99/47069, US 2002/143302, US 2014/194776, US 2006/189940. The technical problem to be solved is to provide such a device which would be an independent element collaborating with the urethro-cystoscope, fit for sterilization in case of making it of surgical steel or relatively cheap to produce from plastic as disposable device, improving the precision of fluid injections into the tissue walls, in particular into the urethra walls. Furthermore, such device shall make it possible to perform radial punctures of the urethra walls at an angle, with precisely set angle and with control of puncture depth, and with visual control of needle application point. Unexpectedly, the problems mentioned above have been solved by the present invention. The first object of this invention is an apparatus for performance of injections at an angle into the walls of the urethra to a predetermined depth, comprising an urethro-cytoscope with a visual inspection system and an injection needle, characterized in that it furthermore comprises a urethro-cytoscope needle guide (11) comprising a rigid tubular member (1) with a first (2) and a second ending (3), wherein the tubular member (1) has at least one longitudinal aperture (4) on its surface, parallel to the longitudinal axis of the tubular member (1), wherein the guide and the urethro-cytoscope are configured such that during the performance of injections at an angle, the urethro-cytoscope (17) is supported on a first support point (8) located on the opening of the second ending of the tubular member and on a second support point (5) or (10) located at a distance (L) from the second ending of the tubular member along the longitudinal axis of the guide, and such that the urethro-cytoscope needle extends outside the guide (11) through the longitudinal aperture (4). In further embodiments of the invention, the internal diameter of the tubular member changes in a conical or non-linear way. Preferably, the distance from the second end of the tubular member to the speculum tool support point, defined by the second end of the longitudinal aperture or the cross- beam, ranges from 5 to 50 mm. Equally preferably, the first end of the longitudinal aperture and/or the cross-beam/beams have cut-offs. In another preferred embodiment of the invention the guide is provided with a removable elastic leash situated in the area of the second end of the guide. Preferably, the leash is made of ethane polymer. In another preferred embodiment of the invention, the application guide is placed on a flexible or rigid application rod, on which it is locked on the stepped, conical, or non-linear change of the internal diameter of the tubular member.

Exemplary embodiments of the invention have been presented in the drawings, where fig. 1 shows the guide according to the first embodiment of the invention, with the urethro-cystoscope attached, in isometric view, fig. 2 shows the guide according to the first embodiment of the invention, with the urethro-cystoscope attached, in side view, fig. 3 shows the guide according to the second embodiment of the invention in isometric view, fig. 4 shows longitudinal cross-section of the guide according to the second embodiment of the invention, fig. 5 shows the guide according to the second embodiment of the invention, with the urethro-cystoscope attached, in side view, fig. 6 shows the guide according to the second embodiment of the invention in isometric view with the leash attached, fig. 7 shows the guide according to the second embodiment of the invention in isometric view placed on the rod, fig. 8 shows the needle lock in isometric view, fig. 9 shows the apparatus for injections at an angle with the guide according to the second embodiment of the invention, in isometric view, with the leash attached and with the needle lock, and fig. 10 shows the apparatus for injections at an angle with the guide according to the second embodiment of the invention, in side view, with the leash attached and with the needle lock.

### Example 1

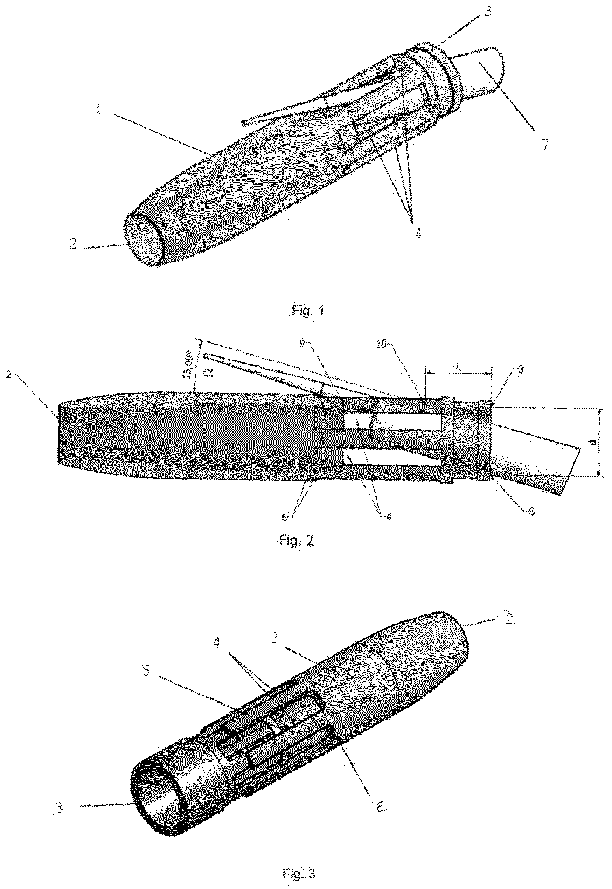

Guide 11 for speculum for injections at an angle according to the first embodiment of the invention, illustrated in isometric and lateral view in fig. 1 and fig. 2, respectively, comprises a rigid tubular element 1 with a first end 2 and a second end 3.The guide is adapted for working with urethro-cystoscope 17,in particular for injection of treatment agent around the urethra in which it is placed. The guide 11 is made of AISI 316 (EN 1.4401) steel and is suitable for sterilization and may be used many times. On the surface of the guide 11, there are six radially arranged longitudinal apertures 4, parallel to the longitudinal axis of the tubular member 1. The width of the apertures enables free passage of urethro-cystoscope needle 17.The first end of the longitudinal aperture 6 has a cut-off which improves the stability of the angular placement of the urethro-cystoscope 17 inside the guide after moving the needle 11 out. According to the first embodiment of the invention, the guide 11 becomes slightly narrower at the first end 2, which gives it a streamlined shape facilitating application. The Internal diameter of the tubular member 1that the guide 11 is made, changes in steps, being smaller at the first ending 2 than at the second ending 3.Stepped change of the internal diameter d of the tubular member 1 provides locking the application rod 13 used for application of the guide 11. Appropriate selection of geometrical parameters of the guide 11 components ensures obtaining the predetermined needle application angle and injection depth. For example, selecting the internal diameter d of the tubular member 1 at the second ending 3 of the tubular member, which constitutes the first support point 8, to be 6 mm and the distance L from the second ending 2 of the tubular member to the support point 10 of the speculum, determined by the second end of the longitudinal aperture 6, being 22 mm, the needle application angle α of 15° is obtained. The α angle value is calculated from simple geometrical relations, where tg(α)=d/L. The structure of the urethro-cystoscope 17 provides locking the tool in the guide 11 always at two support points, 8 and 10,which provides repeatable performance of injection. Furthermore, radially arranged longitudinal apertures 4 provide the view of the place of application of the treatment agent through a visual system of the urethro-cystoscope 17 and enable performing injections around the body in which the guide is situated, in this case - the urethra.

### Example 2

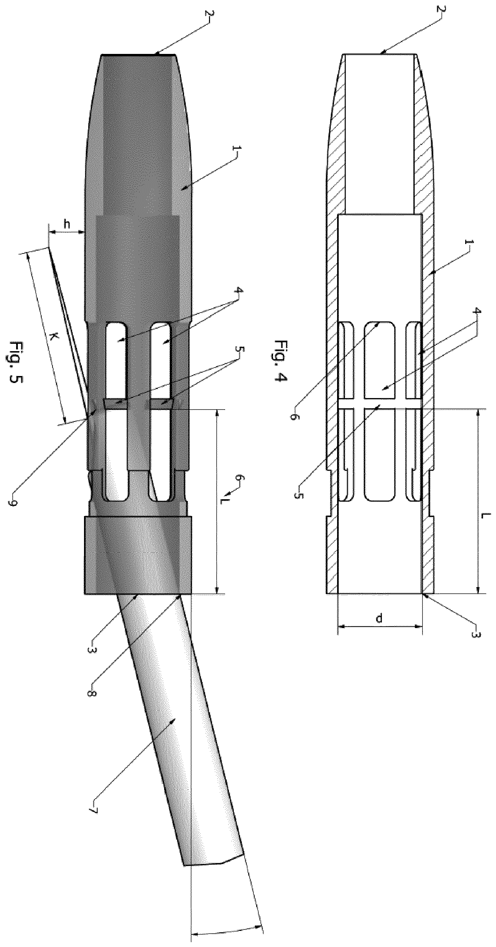

Fig. 3 - 5 show the endoscope needle guide 11 for injections at an angle according to the second embodiment of the invention. The design of the guide 11 is analogous to the design of the guide 11 from the first embodiment of the invention, the difference being that the radially arranged longitudinal apertures 4 comprise a cross-beam 5, dividing those longitudinal apertures 2 into two parts. The application of the cross-beam 5 enables more stable positioning of the urethro-cystoscope and provides better preview of the injection place through the inspection head as compared to the version without the cross-beam. Using the cross-beam 5 as the first support point and the ending 8 as the second support point, a constant needle application angle α is achieved, for example 14°,where tg(α)=d/L. The cross-beam 5 has a cut-off which facilitates guiding the endoscope needle and improves the visibility of the placement for the urethro-cystoscope optical system 17 within the guide 11.Like in the previous example, knowing the application angle and the urethro-cystoscope 17 needle movement distance, it is possible to determine,with simple geometrical dependencies, the injection depth h. For example, knowing the distance K by which the needle extends from the guide, and the needle application angle α, the injection depth h can be calculated using the relation where h = K*sin(α). Therefore, the change of the puncture depth h for the given guide can be obtained by changing the needle lock, which affects the length K by which the needle extends from the endoscope. If desired, it is possible to select other values of d and L, obtaining another injection angle α.

### Example 3

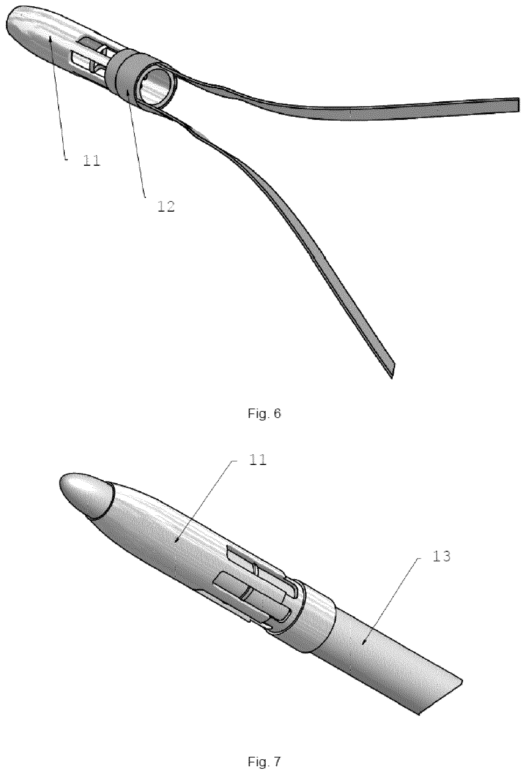

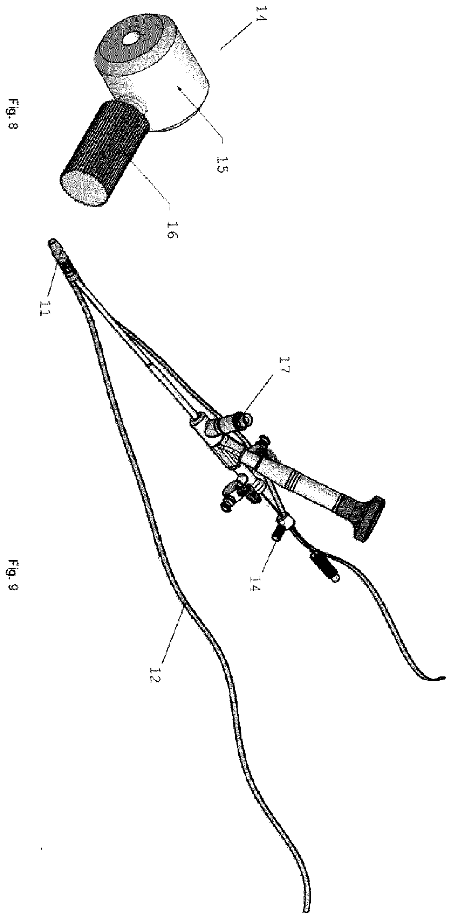

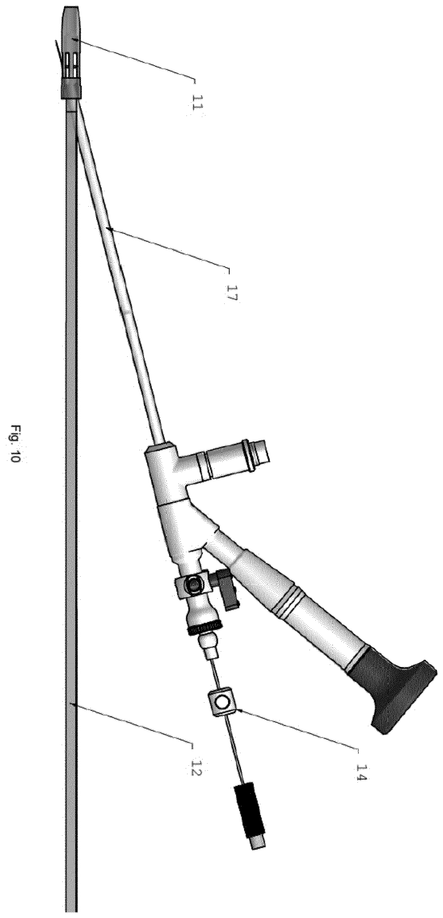

The guide 11 for the speculum, illustrated in example 2,is equipped with removable flexible leash 12 made of ethane polymer, as illustrated in fig. 6.The flexible leash 12 is placed in the area of the second end 3 of the guide 11. The leash 12 has elongated bands, running outside through the body in which the guide 11 is situated. The function of the leash 12 is to enable removal of guide 11 after the desired treatment has been performed, in this case - injection in the urethra regions. In order to apply the guide 11 in the appropriate place, the rod 13 is used, as illustrated in fig. 7.The rod 13 is locked by the stepped change of the internal diameter d of the tubular member 1,which enables moving it into the desired position. The larger internal diameter d of the tubular member 1 at the opposite end of the guide 11 enables free removal of the flexible rod 13 after the application of the guide 11 in the appropriate place. Having introduced the guide 11 in the proper place, e.g. the urethra region, and after removing the flexible rod 13, the urethro-cystoscope 17 is introduced into the body, equipped with a visual inspection system enabling the identification of the guide 11 application place. The urethro-cystoscope 17 positioned in the guide 11 with the leash 12 and needle lock 14 mounted is illustrated in fig. 9 and fig. 10.The needle of the urethro-cystoscope 17 passes through the longitudinal aperture 4 of the guide 11, its maximum extension beyond the contour of the guide 11 being provided by the design of the urethro-cystoscope 17 itself, which is supported in two support points 5 and 8 according to the embodiment with the cross-beam and by using the needle lock 14 which prevents further extension of the needle. The needle lock is situated in the area opposite to the application end of the needle and its positioning can be changed, and by the same adjusted to the specific case. The needle lock illustrated in fig. 8 consists of a locking cylinder 15 and a thumb screw, which makes it a simple structure, easy and cheap in production, and its application does not require the use of any specialized tools.The presented guide 11, together with the needle lock 14, enables injection of the treatment agent around the tissue in which it is placed, preserving exactly the same injection depth.

## Claims

1. Apparatus for performance of injections at an angle into the walls of urethra to the predetermined depth, comprising an urethro-cytoscope (17) with a visual inspection system and an injection needle,
**characterized in that** it furthermore comprises a urethro-cytoscope needle guide (11) comprising a rigid tubular member (1) with a first (2) and a second ending (3), wherein the tubular member (1) has at least one longitudinal aperture (4) on its surface, parallel to the longitudinal axis of the tubular member (1), wherein the guide and the urethro-cytoscope are configured such that during the performance of injections at an angle, the urethro-cytoscope (17) is supported on a first support point (8) located on the opening of the second ending of the tubular member and on a second support point (5) or (10) located at a distance (L) from the second ending of the tubular member along the longitudinal axis of the guide, and such that the urethro-cytoscope needle extends outside the guide (11) through the longitudinal aperture (4).

2. The apparatus according to claim 1, **characterized in that** the tubular member (1) has six radially arranged longitudinal apertures (4) on its surface.

3. The apparatus according to claim 1 or 2, **characterized in that** the longitudinal aperture (4) comprises at least one cross-beam (5).

4. The apparatus according to any of claims from 1 through 3, **characterized in that** the internal diameter (D) of the tubular member (1) decreases moderately at the first end (2) of the tubular member (1).

5. The apparatus according to any of claims from 1 through 4, **characterized in that** it is made of material suitable for sterilization.

6. The apparatus according to any of claims from 1 through 5, **characterized in that** the internal diameter (d) of the tubular member (1) changes discretely or conically, or nonlinearly.

7. The apparatus according to claim 6, **characterized in that** the internal diameter (d) of the tubular member (1) at the first end (2) is smaller than the internal diameter (d) of the tubular member (1) at the second end (3).

8. The apparatus according to any of claims from 1 to 7, **characterized in that** the internal diameter (d) of the tubular member (1) at the second end (3) ranges from 3 to 20 mm.

9. The apparatus according to any of claims from 1 through 8, **characterized in that** the distance L from the second end (3) of the tubular member (1) to the point, defined by the second end of the longitudinal aperture (6) or the cross-beam (5), ranges from 5 to 40 mm.

10. The apparatus according to any of claims from 3 through 9, **characterized in that** the first end of the longitudinal aperture (6) and/or the cross-beam (5) have cut-offs facilitating sliding the needle outside the guide (11).

11. The apparatus according to any of claims from 1 through 10, **characterized in that** is equipped with a detachable flexible leash (12) situated in the area of the second end (3) of the guide (11).

12. The apparatus according to claim 11, **characterized in that** the leash (12) is made of ethane polymer.

13. Apparatus according to claim 12, **characterized in that** it furthermore comprises a needle lock (14) mounted on the urethro-cystoscope needle in the area opposite to the application end of the needle.

## Patentansprüche

1. Vorrichtung zur Durchführung von Injektionen in einem Winkel in die Wände der Ureteren bis zu einer vorbestimmten Tiefe, umfassend ein Urethroskop (17) mit einem visuellen Inspektionssystem und einer Injektionsnadel,
**dadurch gekennzeichnet, dass** es weiterhin eine Urethroskop-Nadelführung (11) umfasst, umfassend ein festes röhrenförmiges Teil (1) mit einem ersten (2) und einem zweiten Ende (3), wobei das röhrenförmige Teil (1) mindestens eine längliche Öffnung (4) auf seiner Oberfläche aufweist, parallel zu der Längsachse des röhrenförmigen Teils (1), wobei die Führung und das Urethroskop so angeordnet sind, dass das Urethroskop (17) während der Durchführung von Injektionen in einem Winkel an einem ersten Tragepunkt (8) getragen wird, der an der Öffnung des zweiten Endes des röhrenförmigen Teils lokalisiert ist, und an einem zweiten Tragepunkt (5) oder (10), der in einem Abstand (L) von dem zweiten Ende des röhrenförmigen Teils entlang der Längsachse der Führung lokalisiert ist, und so, dass sich die Urethroskop-Nadel außerhalb der Führung (11) durch die längliche Öffnung (4) erstreckt.

2. Vorrichtung nach Anspruch 1, **dadurch gekennzeichnet, dass dadurch gekennzeichnet, dass** das röhrenförmige Teil (1) sechs radial angeordnete längliche Öffnungen (4) auf seiner Oberfläche aufweist.

3. Vorrichtung nach Anspruch 1 oder 2, **dadurch gekennzeichnet, dass** die längliche Öffnung (4) mindestens eine Querverstrebung (5) umfasst.

4. Vorrichtung nach einem der Ansprüche von 1 bis 3, **dadurch gekennzeichnet, dass** der innere Durchmesser (D) des röhrenförmigen Teils (1) am ersten Ende (2) des röhrenförmigen Teils (1) moderat verringert ist.

5. Vorrichtung nach einem der Ansprüche von 1 bis 4, **dadurch gekennzeichnet, dass** sie aus einem Material hergestellt ist, das zur Sterilisation geeignet ist.

6. Vorrichtung nach einem der Ansprüche von 1 bis 5, **dadurch gekennzeichnet, dass** sich der innere Durchmesser (d) des röhrenförmigen Teils (1) diskret oder konisch oder nicht-linear verändert.

7. Vorrichtung nach Anspruch 6, **dadurch gekennzeichnet, dass** der innere Durchmesser (d) des röhrenförmigen Teils (1) am ersten Ende (2) kleiner ist als der innere Durchmesser (d) des röhrenförmigen Teils (1) am zweiten Ende (3).

8. Vorrichtung nach einem der Ansprüche von 1 bis 7, **dadurch gekennzeichnet, dass** der innere Durchmesser (d) des röhrenförmigen Teils (1) am zweiten Ende (3) von 3 bis 28 mm reicht.

9. Vorrichtung nach einem der Ansprüche von 1 bis 8 **dadurch gekennzeichnet, dass** der Abstand L von dem zweiten Ende (3) des röhrenförmigen Teils (1) zu dem Punkt, definiert durch das zweite Ende der länglichen Öffnung (6) oder der Querverstrebung (5), von 5 bis 48 mm reicht.

10. Vorrichtung nach einem der Ansprüche von 1 bis 9, **dadurch gekennzeichnet, dass** das erste Ende der länglichen Öffnung (6) und/oder die Querverstrebung (5) Aussparungen haben, die das Herausgleiten der Nadel außerhalb der Führung (11) ermöglichen.

11. Vorrichtung nach einem der Ansprüche von 1 bis 10, **dadurch gekennzeichnet, dass** sie mit einer abnehmbaren flexiblen Lasche (12) ausgerüstet ist, die im Bereich des zweiten Endes (3) der Führung (11) angeordnet ist.

12. Vorrichtung nach Anspruch 11, **dadurch gekennzeichnet, dass** die Lasche (12) aus Ethanpolymer hergestellt ist.

13. Vorrichtung nach Anspruch 12, **dadurch gekennzeichnet, dass** sie weiterhin eine Nadelsperre (14) umfasst, die auf der Urethroskop-Nadel im Bereich gegenüber des Anwendungsendes der Nadel montiert ist.

## Revendications

1. Appareil pour la réalisation d'injections à un angle dans les parois de l'urètre à une profondeur prédéterminée, comprenant un urétro-cytoscope (17) doté d'un système d'inspection visuel et d'une aiguille d'injection,
**caractérisé en ce qu'**il comprend en outre un guide d'aiguille d'urétro-cytoscope (11) comprenant un élément tubulaire rigide (1) doté d'une première (2) et d'une deuxième terminaison (3), dans lequel l'élément tubulaire (1) a au moins un orifice longitudinal (4) sur sa surface, parallèle à l'axe longitudinal de l'élément tubulaire (1), dans lequel le guide et l'urétro-cytoscope sont configurés de telle sorte que pendant la réalisation d'injections à un angle, l'urétro-cytoscope (17) est supporté sur un premier point de support (8) situé sur l'ouverture de la deuxième terminaison de l'élément tubulaire et sur un deuxième point de support (5) ou (10) situé à une distance (L) de la deuxième terminaison de l'élément tubulaire le long de l'axe longitudinal du guide, et de telle sorte que l'aiguille d'urétro-cytoscope s'étende à l'extérieur du guide (11) au travers de l'orifice longitudinal (4).

2. Appareil selon la revendication 1, **caractérisé en ce que** l'élément tubulaire (1) a six orifices longitudinaux (4) agencés radialement sur sa surface.

3. Appareil selon la revendication 1 ou 2, **caractérisé en ce que** l'orifice longitudinal (4) comprend au moins une barre transversale (5).

4. Appareil selon l'une quelconque des revendications 1 à 3, **caractérisé en ce que** le diamètre interne (D) de l'élément tubulaire (1) diminue modérément à la première extrémité (2) de l'élément tubulaire (1).

5. Appareil selon l'une quelconque des revendications 1 à 4, **caractérisé en ce qu'**il est constitué d'un matériau approprié pour la stérilisation.

6. Appareil selon l'une quelconque des revendications 1 à 5, **caractérisé en ce que** le diamètre interne (d) de l'élément tubulaire (1) change de façon discrète ou conique, ou non linéaire.

7. Appareil selon la revendication 6,
**caractérisé en ce que** le diamètre interne (d) de l'élément tubulaire (1) à la première extrémité (2) est plus petit que le diamètre interne (d) de l'élément tubulaire (1) à la deuxième extrémité (3).

8. Appareil selon l'une quelconque des revendications 1 à 7, **caractérisé en ce que** le diamètre interne (d) de l'élément tubulaire (1) à la deuxième extrémité (3) va de 3 à 20 mm.

9. Appareil selon l'une quelconque des revendications 1 à 8, **caractérisé en ce que** la distance L de la deuxième extrémité (3) de l'élément tubulaire (1) au point, défini par la deuxième extrémité de l'orifice longitudinal (6) ou la barre transversale (5), va de 5 à 40 mm.

10. Appareil selon l'une quelconque des revendications 3 à 9,
**caractérisé en ce que** la première extrémité de l'orifice longitudinal (6) et/ou la barre transversale (5) ont des découpes facilitant le glissement de l'aiguille à l'extérieur du guide (11).

11. Appareil selon l'une quelconque des revendications 1 à 10,
**caractérisé en ce qu'**il est équipé d'une bride flexible détachable (12) située dans la zone de la deuxième extrémité (3) du guide (11).

12. Appareil selon la revendication 11,
**caractérisé en ce que** la bride (12) est constituée de polymère d'éthane.

13. Appareil selon la revendication 12,
**caractérisé en ce qu'**il comprend en outre un verrouillage d'aiguille (14) monté sur l'aiguille d'urétro-cytoscope dans la zone opposée à l'extrémité d'application de l'aiguille.
